# EUROPEAN PATENT SPECIFICATION

(11) **EP 3 815 677 B1**
(45) Date of publication and mention of the grant of the patent: **30.08.2023**
(21) Application number: 20204374.1
(22) Date of filing: 28.10.2020
(51) Int. Cl.: A61K 9/08, A61K 47/18, A61K 31/365, A61K 31/4439, A61P 33/10, A61P 33/14

(54) **STABLE VETERINARY COMPOSITION COMPRISING MOXIDECTIN AND IMIDACLOPRID**
STABILE VETERINÄRMEDIZINISCHE ZUSAMMENSETZUNG MIT MOXIDECTIN UND IMIDACLOPRID
COMPOSITION VÉTÉRINAIRE STABLE COMPRENANT DE LA MOXIDECTINE ET DE L'IMIDACLOPRIDE

(30) Priority: 30.10.2019 EP 19206227
(43) Date of publication of application: 05.05.2021
(73) Proprietor: KRKA, d.d., Novo mesto, 8501 Novo mesto (SI)
(72) Inventor: TURK, Urska, 8000 Novo mesto (SI); TIHI, Jaroslav, 8000 Novo mesto (SI); LESKOVAR, Denise, 2317 Oplotnica (SI); MAV GOLEZ , Ida, 1235 Radomlje (SI); ZIBERT, Tanja, 1360 Vrhnika (SI); STERGAR, Matej, 1000 Ljubljana (SI); URSIC, Darko, 1292 Ig (SI)
(74) Representative: Kutzenberger Wolff & Partner

(56) References cited:
- WO-A1-2018/029487
- WO-A1-2019/101971
- US-A1- 2012 071 484
- US-A1- 2015 209 355

## Description

This application claims priority to European patent application no. 19 206 227.1 which was filed on October 30, 2019.

The invention relates to a stabilized veterinary composition comprising Moxidectin in combination with Imidacloprid and an amine base for cutaneous topical application (spot-on), to the use of an amine base for stabilizing Moxidectin, and to the use of an amine base for producing a veterinary composition with improved Moxidectin stability.

Many animals, especially pets are at risk of or suffer from parasitic infections, especially mixed parasitic infections. Several veterinary compositions have therefore been developed that are said to be useful against parasitic infections in animals without providing significant harm to humans. Many of these veterinary compositions contain a combination of two or more antiparasitic ingredients.

WO 96/17520 relates to compositions for the dermal control of parasitic insects on animals, containing agonists or antagonists of the nicotinergic acetylcholine receptors of insects, solvents from the group benzyl alcohol or optionally substituted pyrrolidones, solvents from the group of cyclic carbonates or lactones and optionally further auxiliaries.

WO 98/27817 relates to the use of avermectin B1a /B1b, 22,23-dihydroavermectin B1a /B1b, doramectin or moxidectin with imidacloprid, Ti 435 or AKD 1022; optionally further active ingredients and diluents or carriers, for the preparation of compositions for dermal application for combatting of ecto- and endoparasites in a single treatment.

WO 99/41986 discloses water-containing formulations for the dermal control of parasitic insects on animals containing imidacloprid, 2.5 to 15% by weight of water, solvents from the group alcohols or optionally substituted pyrrolidones, solvents from the group of the cyclic carbonates or lactones, if desired, further auxiliaries and further active compounds.

US 2002/177597 discloses a synergistic insecticidal composition comprising as essential active ingredients a neuronal sodium channel antagonist in combination with one or more compounds selected from the group consisting of pyrethroids, pyrethroid-type compounds, recombinant nucleopolyhe-droviruses capable of expressing an insect toxin, organophosphates, carbamates, formamidines, macrocyclic lactones, amidinohydrazones, GABA antagonists and acetylcholine receptor ligands.

US 2003/166688 relates to spot-on compositions for the treatment or prophylaxis of parasite infestations in mammals or birds which comprise: (1) a composition comprising (A) an effective amount of a 1-phenylpyrazole derivative; and (B) an effective amount of Emamectin; (2) an acceptable liquid carrier vehicle; and (3) optionally, a crystallization inhibitor.

WO 2005/074912 provides an antiparasitic composition comprising an anti-parasitically effective amount of an organic amine salt of Closantel, optionally a macrocyclic lactone and a non-irritating solvent system consisting essentially of an alcohol and a glycol derivative. The document is silent on Imidacloprid.

WO 2006/050816 relates to the use of macrocyclic lactones for treating demodicosis. The example 7 (100 mg/25 mg/ml) and example 8 (100 mg/10 mg/ml) correspond to the marketed reference product Advocate^{®} spot-on solution.

US 2009/0036458 relates to a composition which comprises: an effective amount of (a) Praziquantel and (b) a second endoparasiticidal agent selected from the group consisting of a macrocyclic lactone, Imidacloprid and a combination thereof; and (c) 4-allyl-2-methoxyphenol as carrier.

US 2011/0160218 relates to formulations which may comprise macrocyclic lactones and at least one spirodioxepinoindole derivative or a spirooxepinoindole derivative for combating parasites in birds and mammals.

US 2012/071484 relates to a topical parasiticide composition comprising: (i) a phenyl pyrazole insecticide; and/or a neonicotinoid; (ii) a macrocylic lactone and/or an aminoacetonitrile derivatives; (iii) an Insect Growth Regulator; and (iv) a 2-acyl-4-oxo-1,2,3,6,7,11b-4H-pyrazino[2,1a]isoquinoline derivative. While exemplified formulations contain Moxidectin, Imidacloprid and as a solvent 2-pyrrolidone (lactam, pK_{A} >10.00), the document is silent on amine bases, let alone any stabilizing effect thereof.

CN 106 511 267 provides compound Moxidectin drops as well as a preparation method and application thereof. The compound Moxidectin drops have the advantages that Moxidectin and ingredients with insecticidal effect, such as imidacloprid, are compounded and used, so that the drops have good insecticidal effect; meanwhile, in the drops, propylene glycol, moringa seed oil and eucalyptus oil are taken as transdermal agents, wherein the moringa seed oil and the eucalyptus oil are natural plant essential oil with fragrance and can cover up peculiar smell in the drops and improve sense of a user; and further the drops can be used for killing endoparasite and ectoparasite of animals due to good insecticidal effect. While exemplified formulations contain Moxidectin, Imidacloprid and as a solvent 2-pyrrolidone (lactam, pK_{A} >10.00), the document is silent on amine bases, let alone any stabilizing effect thereof.

US 2013/0225516 relates to stable, highly-effective topical formulations comprising Permethrin, Fipronil and a solvent system that is sufficient to solubilize these two active ingredients and limit degradation of Fipronil to its sulfone, and their uses in topical applications on animals and the environment.

WO 2014/169092 is directed to stabilized compositions comprising at least one macrocyclic lactone, or derivative thereof, in combination with levamisole, and an amino sugar stabilizing agent, optionally an additional antiparasitic agent, and a method for treating or controlling a parasitic infection or infestation in an animal by administering said composition. The document is silent on Imidacloprid. An exemplified composition containing Moxidectin and 0.025 %w/w meglumine after storage 12 weeks at 40°C only contains 92.0 %w/w of the originally contained amount of Moxidectin prior to storage, i.e. storage leads to a Moxidectin degradation by 8.0 %w/w.

US 2019/70158 relates to a monodose veterinary composition for topical application (spot-on) for canines, the said composition comprising: (a) Imidacloprid, between 8.18 and 10.00 %, (b) Moxidectin, between 2.86 and 3.50%, (c) Praziquantel, between 8.18 and 10.00%, and (d) an aprotic solvent selected from dimethyl sulfoxide (DMSO), N,N-dimethylacetamide (DMAC), N,N-dimethylformamide (DMF) and their mixtures, between 65.45 and 80.00 %; together with excipients acceptable from the veterinary viewpoint, where the percentages are related to the composition's total weight.

WO 2019/101971 relates to a veterinary or pharmaceutical composition comprising: (i) about 1-65 % w/v of a pyrethroid or a salt thereof, (ii) a macrocyclic lactone or a salt thereof, (iii) at least one alkalizing agent, (iv) at least one non aqueous solvent, wherein the pH of the composition is comprised between about 6.5 and 8.5, when measured by adding 25% of water to an aliquot of said composition. The amount of alkalizing agent, preferably sodium hydroxide, depends on the permethrin final grade, permethrin purity grade and permethrin acidity. The document is silent on Imidacloprid.

Imidacloprid and Moxidectin are both well-established antiparasiticides with proven efficacy. Veterinary compositions comprising both Imidacloprid and Moxidectin are difficult to formulate, as the desired routes of administration are different (Moxidectin transdermally/systemically; Imidacloprid intradermally/locally).

A veterinary composition comprising a combination of Imidacloprid and Moxidectin for spot-on application is commercially available (Advocate^{®}, Bayer Animal Health). This combination is an endectocide, i.e. is effective against both, ecto- and endoparasites, like fleas, lice, mites and different types of worms (see e.g. C. Le Sueur et al., Parasitol Res. 2010 Nov;107(6):1463-9. doi: 10.1007/ s00436-010-2022-8; R.D. Paran et al., Vet Med Int. 2011;2011:482746. doi: 10.4061/2011/482746; F. Veronesi et al., Vet Parasitol. 2014 Feb 24;200(1-2):133-8. doi: 10.1016/j.vetpar.2013.11.026; J.J. Fourie et al., Parasit Vectors. 2015 Mar 28;8: 187. doi: 10.1186/s13071-015-0775-8; D.D. Bowman et al., Parasit Vectors. 2017; 10(Suppl 2): 478, doi: 10.1186/s13071-017-2432-x; D. Otranto et al., Vet Parasitol. 2016 Aug 30;227:118-21. doi: 10.1016/j.vetpar.2016.07.035; D. Otranto et al., Parasit Vectors. 2019 Jan 11;12(1):25. doi: 10.1186/s13071-018-3262-1; and G. Segev et al., Parasit Vectors. 2018 Mar 5;11(1):127. doi: 10.1186/s13071-018-2731-x).

Moxidectin penetrates through the skin, enters the bloodstream, and distributes in all organs. It acts systemically, targeting endo-parasites and mites. Imidacloprid spreads in the water resistant lipid layer of the skin's surface and acts topically, targeting ectoparasites. The commercial product is monthly applied spot-on building up a plateau of antiparasitic Moxidectin for continuous and long-lasting protection against internal parasites. The product is waterproof, i.e. remains effective even if the animal becomes wet. Advocate^{®} is marketed in polypropylene unit dose pipettes with screw cap. The indicated shelf-life of the commercial product as packaged for sale is 3 years. However, special precautions for storage need to be taken; Advocate^{®} should not be stored above 30 °C (in accordance with EMEA/CVMP/422/99/Rev.3 (December 2007): *Guideline on Declaration of Storage Conditions in the Product Information of the Pharmaceutical veterinary Medicinal Products and for Active Substance).*

The properties of the known veterinary compositions that contain Moxidectin and Imidacloprid and that are useful for treating mixed parasitic infections in animals are not satisfactory in every respect. In particular, as already mentioned above, known veterinary compositions containing Moxidectin and Imidacloprid such as Advocate^{®} have limited storage stability and therefore require specific storage conditions.

There is therefore a demand for veterinary compositions comprising Moxidectin in combination with Imidacloprid that have improved storage stability so that no specific storage conditions need to be met and need to be mentioned in the product information, respectively. The veterinary compositions should ensure that Moxidectin penetrates through the skin, enters the bloodstream, and distributes in all organs, i.e. acts systemically, targeting endo-parasites and mites; whereas Imidacloprid spreads in the water resistant lipid layer of the skin's surface and acts topically, targeting ectoparasites.

It is an object of the invention to provide veterinary compositions comprising Moxidectin in combination with Imidacloprid that have advantages compared to the prior art. The veterinary compositions should have excellent storage stability and at the same time should provide the desired antiparasitic effect against both, ecto- and endoparasites based upon the desired route of administration (Moxidectin transdermally/systemically; Imidacloprid intradermally/locally). The veterinary compositions should be more stable and contain less degradation products/impurities than the already marketed originator product Advocate^{®}.

This object has been achieved by the subject-matter of the patent claims.

Extensive research revealed that in liquid veterinary compositions Moxidectin is prone to degradation. Two degradation products of Moxidectin have been evaluated in finished drug product, the isomer of Moxidectin that is formed due to oxidation of Moxidectin, and 23-keto-nemadectin, a hydrolytic degradation product. It has been surprisingly found that Moxidectin can be stabilized against decomposition into these degradation products by means of an amine base at comparatively low concentrations.

Further, it has been found that Imidacloprid is compatible with such amine base. Still further, it has been found that the presence of amine base does not significantly alter the two different desired routes of administration (Moxidectin transdermally/systemically; Imidacloprid intradermally/locally).

Based upon the results of accelerated stability studies performed at 40°C/75%RH for 6 months, the veterinary composition according to the invention does not require any special temperature storage conditions (according to EMEA/CVMP/422/99/Rev.3 (December 2007): *Guideline on Declaration of Storage Conditions in the Product Information of the Pharmaceutical veterinary Medicinal Products and for Active Substance*), whereas the marketed originator product Advocate^{®} according to the currently valid summary of product characteristics (SmPC) should not be stored above 30 °C.

Without wishing to be bound to any scientific theory, it appears that certain amine bases, especially comparatively weak amine bases having pK_{A} values of the corresponding protonated forms (conjugate acids) within the range of from about 7.2 to about 10.0, are capable of stabilizing Moxidectin against chemical decomposition in veterinary compositions additionally containing Imidacloprid.

A first aspect of the invention relates to a veterinary composition for cutaneous topical application comprising
(i) Moxidectin or a physiologically acceptable salt thereof;
(ii) Imidacloprid or a physiologically acceptable salt thereof; and
(iii) a physiologically acceptable amine base selected from the group consisting of triethanolamine, diethanolamine, monoethanolamine, N-methyldiethanolamine, N-ethyldiethanolamine, N,N-dimethylethanolamine, and N,N-diethylethanolamine; wherein the weight content of the amine base is within the range of from 1 ppm to 1000 ppm, relative to the total weight of the veterinary composition.

The composition according to the invention is a veterinary composition that is suitable and typically also devoted for the prevention and/or the treatment of parasitic infections, particularly mixed parasitic infections, in animals, preferably in pets, more preferably in mammalian pets, most preferably in dogs, cats and ferrets.

The veterinary composition according to the invention is suitable and typically also devoted for cutaneous topical application, preferably as a spot-on composition, more preferably as a spot-on solution.

For the purpose of the specification, a spot-on composition is a dosage form that is to be applied to the skin of the animal. Preferably, the spot-on composition is liquid and is provided e.g. in single-use pipettes or other dispensing devices containing a defined volume of the veterinary composition and containing the entire dose to be administered all at once. Depending upon the size and weight of the animal to be treated, the spot-on composition is to be applied, e.g. pipetted, to a single spot of skin or to several spots of skin, typically to 1, 2, 3 or 4 spots. Whenever possible, the skin should be undamaged skin of the animal. When being provided in a pipette, the tip of the pipette may be tipped on the skin of the animal and may be squeezed firmly several times to empty its content directly onto the skin, i.e. to a single spot or to several spots. Application of the spot-on composition at the base of the skull, between the shoulder blades, along the top of the back from between the shoulders to the base of the tail, and the like, minimizes the opportunity for the animal to lick the veterinary composition.

The veterinary composition according to the invention comprises Moxidectin or a physiologically acceptable salt thereof.

Moxidectin, 23-(O-methyloxime)-F28249 alpha (CAS 113507-06-5, ATCvet code QP54AB02) is a second-generation macrocyclic lactone of the milbemycin family. It is a parasiticide which is active against many internal and external parasites. Moxidectin is active against larval stages (L3, L4) of *Dirofilaria immitis.* It is also active against gastrointestinal nematodes. Moxidectin interacts with GABA and glutamate-gated chloride channels. This leads to opening of the chloride channels on the postsynaptic junction, the inflow of chloride ions and induction of an irreversible resting state. The result is flaccid paralysis of affected parasites, followed by their death and/or expulsion. Moxidectin is a semisynthetic derivative of nemadectin which can be produced by fermentation by *Streptomyces cyanogriseus subsp.noncyanogenus.*

The chemical name of Moxidectin is: (2aE,2'R,4E,4'E,5'S,6R,6'S,8E,11R,15S,17aR,20R, 20aR,20bS)-6'-[(1E)-1,3-Dimethylbut-l-enyl]-20,20b-dihydroxy-4'-(methoxyimino)-5',6,8,19-tetramethyl-3',4',5',6,6',7,10,11,14,15,17a,20,20a,20b-tetradecahydrospiro[2H,17H-11,15-methanofuro-[4,3,2-pq]-[2,6]benzodioxacyclooctadecine-13,2'-pyran]-17-one ((6R,23E,25S)-5O-demethyl-28-de-oxy-25-[(1E)-1,3-dimethylbut-1-enyl]-6,28-epoxy-23-(methoxyimino)milbemycin B). Moxidectin is described in e.g. US 4,916,154. It has the molecular formula C₃₇H₅₃NO₈ and a molecular weight Mr of 640 g/mol. The structure of Moxidectin is depicted here below:

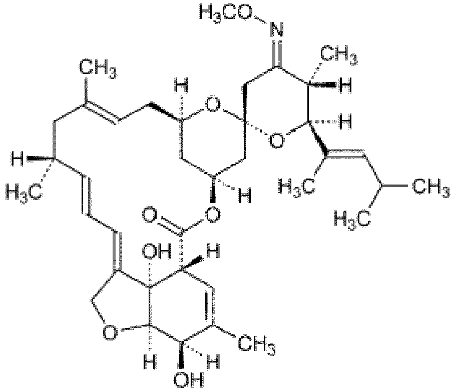

Preferably, Moxidectin is present in a non-salt form. Preferably, upon preparation of the veterinary composition according to the invention, Moxidectin is employed in its non-salt form, whereas it is contemplated that depending upon the chemical environment of the veterinary composition that is influenced by all its excipients, one or more salts of Moxidectin may be formed *in situ.* For the purpose of the specification, unless expressly stated otherwise, all quantities and percentages of Moxidectin are expressed in mg and percent by weight, respectively, and refer to the molecular weight of the non-salt form of Moxidectin. Thus, any potential contributions of salt forming entities are not to be considered when determining quantities and percentages.

In a preferred embodiment, the veterinary composition according to the invention is a liquid and contains at least 4.0 mg/ml of Moxidectin, preferably in its non-salt form, more preferably at least 5.0 mg/ml, still more preferably at least 6.0 mg/ml, yet more preferably at least 7.0 mg/ml, even more preferably at least 8.0 mg/ml, most preferably at least 9.0 mg/ml, and in particular at least 10.0 mg/ml, in each case relative to the total volume of the veterinary composition. In another preferred embodiment, the veterinary composition according to the invention is a liquid and contains at least 12 mg/ml of Moxidectin, preferably in its non-salt form, more preferably at least 15 mg/ml, still more preferably at least 17 mg/ml, yet more preferably at least 19 mg/ml, even more preferably at least 21 mg/ml, most preferably at least 23 mg/ml, and in particular at least 25 mg/ml, in each case relative to the total volume of the veterinary composition.

In a preferred embodiment, the veterinary composition according to the invention is a liquid and contains at most 25 mg/ml of Moxidectin, preferably in its non-salt form, more preferably at most 20 mg/ml, still more preferably at most 18 mg/ml, yet more preferably at most 16 mg/ml, even more preferably at most 14 mg/ml, most preferably at most 12 mg/ml, and in particular at most 10 mg/ml, in each case relative to the total volume of the veterinary composition. In another preferred embodiment, the veterinary composition according to the invention is a liquid and contains at most 37 mg/ml of Moxidectin, preferably in its non-salt form, more preferably at most 35 mg/ml, still more preferably at most 33 mg/ml, yet more preferably at most 31 mg/ml, even more preferably at most 29 mg/ml, most preferably at most 27 mg/ml, and in particular at most 25 mg/ml, in each case relative to the total volume of the veterinary composition.

Preferably, the veterinary composition according to the invention is a liquid and the content of Moxidectin, preferably in its non-salt form, is within the range of 17.5±17.0 mg/ml of, more preferably of 17.5±16.0 mg/ml, still more preferably of 17.5±15.0 mg/ml, yet more preferably of 17.5±14.0 mg/ml, even more preferably of 17.5± 12.0 mg/ml, most preferably of 17.5± 10.0 mg/ml, and in particular of 17.5±8.0 mg/ml, in each case relative to the total volume of the veterinary composition. In a preferred embodiment, the veterinary composition according to the invention is a liquid and the content of Moxidectin, preferably in its non-salt form, is within the range of 10±7 mg/ml of, more preferably of 10±6 mg/ml, still more preferably of 10±5 mg/ml, yet more preferably of 10±4 mg/ml, even more preferably of 10±3 mg/ml, most preferably of 10±2 mg/ml, and in particular of 10±1 mg/ml, in each case relative to the total volume of the veterinary composition. In another preferred embodiment, the veterinary composition according to the invention is a liquid and the content of Moxidectin, preferably in its non-salt form, is within the range of 25±14 mg/ml of, more preferably of 25±12 mg/ml, still more preferably of 25±10 mg/ml, yet more preferably of 25±8 mg/ml, even more preferably of 25±6 mg/ml, most preferably of 25±4 mg/ml, and in particular of 25±2 mg/ml, in each case relative to the total volume of the veterinary composition.

The veterinary composition according to the invention comprises Imidacloprid or a physiologically acceptable salt thereof.

Imidacloprid, 1-(6-Chloro-3-pyridylmethyl)-N-nitro-imidazolidin-2-ylideneamine (CAS 138261-41-3, ATCvet code QP53AX17) is an ectoparasiticide belonging to the chloronicotinyl group of compounds. Chemically, it is more accurately described as a chloronicotinyl nitroguanidine. Imidacloprid is effective against larval flea stages and adult fleas. Flea larvae in the pet's surroundings are killed after contact with a pet treated with the product. Imidacloprid has a high affinity for the nicotinergic acetylcholine receptors in the post-synaptic region of the central nervous system (CNS) of the flea. The ensuing inhibition of cholinergic transmission in insects results in paralysis and death. Due to the weak nature of the interaction with mammalian nicotinergic receptors and the postulated poor penetration through the blood-brain barrier in mammals, it has virtually no effect on the mammalian CNS. Imidacloprid has minimal pharmacological activity in mammals.

The chemical name of Imidacloprid is N-(1-((6-chloropyridin-3-yl)methyl)imidazolidin-2-ylidene)nitramide. Imidacloprid is described in e.g. US 4,742,060. It has the chemical formula C₉H₁₀ClN₅O₂ and a molecular weight Mr of 255.66 g/mol. The structure of Imidacloprid is depicted here below:

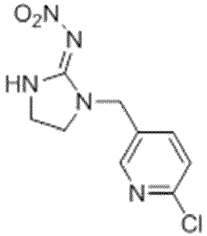

Preferably, Imidacloprid is present in a non-salt form. Preferably, upon preparation of the veterinary composition according to the invention, Imidacloprid is employed in its non-salt form, whereas it is contemplated that depending upon the chemical environment of the veterinary composition that is influenced by all its excipients, one or more salts of Imidacloprid may be formed *in situ.* For the purpose of the specification, unless expressly stated otherwise, all quantities and percentages of Imidacloprid are expressed in mg and percent by weight, respectively, and refer to the molecular weight of the non-salt form of Imidacloprid. Thus, any potential contributions of salt forming entities are not to be considered when determining quantities and percentages.

Preferably, the veterinary composition according to the invention is a liquid and contains at least 50 mg/ml of Imidacloprid, preferably in its non-salt form, more preferably at least 60 mg/ml, still more preferably at least 70 mg/ml, yet more preferably at least 80 mg/ml, even more preferably at least 90 mg/ml, most preferably at least 95 mg/ml, and in particular at least 100 mg/ml, in each case relative to the total volume of the veterinary composition.

Preferably, the veterinary composition according to the invention is a liquid and contains at most 150 mg/ml of Imidacloprid, preferably in its non-salt form, more preferably at most 140 mg/ml, still more preferably at most 130 mg/ml, yet more preferably at most 120 mg/ml, even more preferably at most 110 mg/ml, most preferably at most 105 mg/ml, and in particular at most 100 mg/ml, in each case relative to the total volume of the veterinary composition.

Preferably, the veterinary composition according to the invention is a liquid and the content of Imidacloprid, preferably in its non-salt form, is within the range of 100±70 mg/ml of, more preferably of 100±60 mg/ml, still more preferably of 100±50 mg/ml, yet more preferably of 100±40 mg/ml, even more preferably of 100±30 mg/ml, most preferably of 100±20 mg/ml, and in particular of 100±10 mg/ml, in each case relative to the total volume of the veterinary composition.

While the veterinary composition according to the invention may principally contain additional antiparasitic substances, such as Permethrin, Praziquantel, or Fipronil, it is preferred that Moxidectin and Imidacloprid (and optionally benzyl alcohol) are the only antiparasitic substances that are contained in the veterinary composition.

In preferred embodiments, the veterinary composition according to the invention contains neither Permethrin nor a salt thereof, preferably no pyrethroid at all.

In preferred embodiments, the veterinary composition according to the invention contains neither Closantel (Clozantel) nor a salt thereof.

In preferred embodiments, the veterinary composition according to the invention contains neither Levamisole nor a salt thereof.

In preferred embodiments, the veterinary composition according to the invention contains neither Praziquantel nor a salt thereof.

The veterinary composition according to the invention preferably belongs to the pharmacotherapeutic group of antiparasitic products, insecticides and repellents, macrocyclic lactones, milbemycins.

After topical administration of the veterinary composition, i.e. after topical application to the skin of the animal to be treated, Moxidectin is absorbed through the skin, reaching maximum plasma concentrations preferably about 1 to 2 days after treatment in cats and preferably about 4 to 9 days after treatment in dogs. Following absorption from the skin, Moxidectin is distributed systemically and is slowly eliminated from the plasma. The veterinary composition additionally contains Imidacloprid, which is rapidly distributed over the animal's skin, typically within one day of application.

Therefore, while the veterinary composition according to the invention is for topical cutaneous application, preferably as spot-on composition, the pharmacologically active ingredients Moxidectin and Imidacloprid are administered via different routes. Moxidectin is preferably administered transdermally in order to act systemically, whereas Imidacloprid is preferably administered intradermally in order to act locally within the skin.

Preferably, the veterinary composition according to the invention comprises Moxidectin and Imidacloprid, whereas the relative weight ratio of Imidacloprid to Moxidectin is within the range of from 100:1 to 100:60, more preferably 100:2 to 100:55, still more preferably 100:3 to 100:50, yet more preferably 100:4 to 100:45 , even more preferably 100:5 to 100:40, most preferably 100:6 to 100:35, and in particular 100:7 to 100:30. In a preferred embodiment, the relative weight ratio of Imidacloprid to Moxidectin is within the range of from 100:3 to 100:17, more preferably 100:4 to 100:16, still more preferably 100:5 to 100:15, yet more preferably 100:6 to 100:14 , even more preferably 100:7 to 100:13, most preferably 100:8 to 100:12, and in particular 100:9 to 100:11. In another preferred embodiment, the relative weight ratio of Imidacloprid to Moxidectin is within the range of from 100:18 to 100:32, more preferably 100:19 to 100:31, still more preferably 100:20 to 100:30, yet more preferably 100:21 to 100:29 , even more preferably 100:22 to 100:28, most preferably 100:23 to 100:27, and in particular 100:24 to 100:26.

The veterinary composition according to the invention comprises a physiologically acceptable amine base selected from the group consisting of triethanolamine, diethanolamine, monoethanolamine, N-methyldiethanolamine, N-ethyldiethanolamine, N,N-dimethylethanolamine, and N,N-diethylethanolamine.

Preferably, upon preparation of the veterinary composition according to the invention, the amine base according to the invention is employed in its non-salt form, whereas it is contemplated that depending upon the chemical environment of the veterinary composition that is influenced by all its excipients, one or more salts of the amine base according to the invention may be formed *in situ.* For the purpose of the specification, unless expressly stated otherwise, all quantities and percentages of the amine base according to the invention are expressed in mg and percent by weight, respectively, and refer to the molecular weight of the non-salt form of the amine base according to the invention. Thus, any potential contributions of salt forming entities are not to be considered when determining quantities and percentages.

The amine base according to the invention may be a primary amine, secondary amine or tertiary amine. Preferably, the amine base according to the invention is selected from aliphatic primary amines, aliphatic secondary amines and aliphatic tertiary amines.

Without wishing to be bound on any scientific theory, it appears that the amine base according to the invention has a stabilizing effect and thus prevents Moxidectin from chemical decomposition upon storage. Thus, the amine base according to the invention improves storage stability of the veterinary composition according to the invention such that it does not require storage at temperatures up to 30°C.

Preferably, the amine base according to the invention is a comparatively weak base. Preferably, the protonated form of the amine base (conjugate acid) has a pK_{A} value within the range of from 7.20 to 10.00, more preferably from 7.20 to 9.50, still more preferably from 7.20 to 9.00, yet more preferably from 7.20 to 8.50. In a particularly preferred embodiment, the amine base according to the invention has a pK_{A} value within the range of 7.76±0.55, more preferably 7.76±0.50, still more preferably 7.76±0.45, yet more preferably 7.76±0.40, even more preferably 7.76±0.35, most preferably 7.76±0.25, and in particular 7.76±0.20.

Amine bases according to the invention and the respective pK_{A} values of their conjugate acids are triethanolamine (pK_{A} 7.76), N-methyldiethanolamine (pK_{A} 8.52), N-ethyldiethanolamine (pK_{A} 8.7), diethanolamine (pK_{A} 8.88), N,N-dimethyl-ethanolamine (pK_{A} 9.23), monoethanolamine (pK_{A} 9.40) and N,N-diethylethanolamine (pK_{A} 9.76).

For the purpose of the specification, unless expressly stated otherwise, the pK_{A} values are preferably to be determined in water at 23°C.

The amine base according to the invention is selected from the group consisting of triethanolamine, diethanolamine, monoethanolamine, N-methyldiethanolamine, N-ethyldiethanolamine, N,N-dimethylethanolamine, and N,N-diethylethanolamine.

Triethanolamine is a particularly preferred amine base according to the invention.

The weight content of the amine base according to the invention, preferably triethanolamine, diethanolamine, monoethanolamine, N-methyldiethanolamine, N-ethyldiethanolamine, N,N-dimethylethanolamine, or N,N-diethylethanolamine, more preferably triethanolamine, is within the range of from 1 ppm to 1000 ppm, relative to the total weight of the veterinary composition. For the purpose of the specification, unless expressly stated otherwise, "ppm" shall be based on weight, i.e. "ppmw".

In preferred embodiments the weight content of the amine base according to the invention, preferably triethanolamine, diethanolamine, monoethanolamine, N-methyldiethanolamine, N-ethyldiethanolamine, N,N-dimethylethanolamine, or N,N-diethylethanolamine, more preferably triethanolamine, is at least 40 ppm, more preferably at least 50 ppm, still more preferably at least 60 ppm, yet more preferably at least 70 ppm, even more preferably at least 80 ppm, most preferably at least 90 ppm, and in particular at least 100 ppm, relative to the total weight of the veterinary composition.

In preferred embodiments the weight content of the amine base according to the invention, triethanolamine, diethanolamine, monoethanolamine, N-methyldiethanolamine, N-ethyldiethanolamine, N,N-dimethylethanolamine, or N,N-diethylethanolamine, preferably triethanolamine, is at most 500 ppm, more preferably at most 400 ppm, still more preferably at most 350 ppm, yet more preferably at most 300 ppm, even more preferably at most 250 ppm, most preferably at most 200 ppm, and in particular at most 150 ppm, relative to the total weight of the veterinary composition.

In preferred embodiments the weight content of the amine base according to the invention, triethanolamine, diethanolamine, monoethanolamine, N-methyldiethanolamine, N-ethyldiethanolamine, N,N-dimethylethanolamine, or N,N-diethylethanolamine, preferably triethanolamine, is at most 240 ppm, more preferably at most 230 ppm, still more preferably at most 220 ppm, yet more preferably at most 210 ppm, even more preferably at most 200 ppm, most preferably at most 190 ppm, and in particular at most 180 ppm, relative to the total weight of the veterinary composition.

Preferably, the weight content of the amine base according to the invention, triethanolamine, diethanolamine, monoethanolamine, N-methyldiethanolamine, N-ethyldiethanolamine, N,N-dimethylethanolamine, or N,N-diethylethanolamine, preferably triethanolamine, is within the range of 100±90 ppm, more preferably 100±80 ppm, still more preferably 100±70 ppm, yet more preferably 100±60 ppm, even more preferably 100±50 ppm, most preferably 100±40 ppm, and in particular 100±30 ppm, relative to the total weight of the veterinary composition.

Preferably, the veterinary composition according to the invention has a defined pH value that is to be determined under the following conditions: (i) when the veterinary composition is an aqueous composition, its pH value is directly determined on the veterinary composition as such; or (ii) when the veterinary composition is an anhydrous composition, its pH value is determined by mixing the anhydrous veterinary composition with pure water in order to obtain a 1% v/v aqueous dispersion (e.g. water suspension) and determining the resultant pH value on the thus obtained mixture. For ease of definition, the following preferred pH values refer to either condition (i) or condition (ii), whereas condition (ii) is preferred according to the invention.

Preferably, the pH value of the veterinary composition is at least 5.3, more preferably at least 5.4, still more preferably at least 5.5, yet more preferably at least 5.6, even more preferably at least 5.7, most preferably at least 5.8, and in particular at least 5.9.

Preferably, the pH value of the veterinary composition is at most 6.8, more preferably at most 6.7, still more preferably at most 6.6, yet more preferably at most 6.5, even more preferably at most 6.4, most preferably at most 6.3, and in particular at most 6.2.

Preferably, the pH value of the veterinary composition is within the range of from 5.7 to 6.4, more preferably from 5.8 to 6.3, still more preferably from 5.9 to 6.2.

Preferably, the veterinary composition according to the invention is a liquid, preferably a solution, suspension or emulsion. Preferably, the veterinary composition according to the invention is anhydrous, i.e. contains less than 2 wt.-% water, preferably not more than 1.5 wt.-%, more preferably not more than 1.0 wt.-%, most preferably not more than 0.5 wt.-%, relative to the total weight of the veterinary composition. Solutions are particularly preferred, especially anhydrous solutions.

Preferably, as a consequence of the stabilizing effect of the amine base according to the invention, the veterinary composition according to the invention has improved storage stability. Preferably, the veterinary composition according to the invention is stable in accordance with EMEA/CVMP/ 422/99/Rev.3 (20 December 2007) *Guideline on Declaration of Storage Conditions in the Product Information of the Pharmaceutical veterinary Medicinal Products and for Active Substance* under testing conditions 25°C/60% RH (long term), 40°C/75% RH (accelerated).

The veterinary composition according to the invention may contain conventional excipients in conventional amounts. Suitable excipients and their amounts are known to the skilled person. In this regard it can be referred to e.g. P.J. Sheskey et al., Handbook of Pharmaceutical Excipients, 8th Revised edition (11. August 2017), Pharmaceutical Press.

Preferably, the veterinary composition according to the invention additionally contains one or more excipients selected from antioxidants, preservatives, solvents, and co-solvents.

In general, there is an overlap of the terms "solvent" and "co-solvent". For the purpose of the specification, a co-solvent is added to a primary solvent in a comparatively small amount to increase the solubility of a poorly-soluble compound, in the present case of Moxidectin and/or Imidacloprid. Thus, when the veterinary composition according to the invention contains a co-solvent, it will also contain a solvent (i.e. a primary solvent), and the amount of the solvent will typically exceed to amount of the co-solvent.

Suitable antioxidants include but are not limited to antioxidants selected from the group consisting of di-tert-butylhydroxytoluene (BHT), butylhydroxyanisole (BHA), alpha-tocopherol, alpha-tocopheryl acetate, ascorbic acid, salts of ascorbic acid, monothioglycerol, phosphorous acid, coniferyl benzoate, nordihydro-guajaretic acid, gallus acid esters, sodium bisulfite, and mixtures thereof. Butylhydroxytoluene, preferably di-tert-butylhydroxytoluene, is a particularly preferred antioxidant according to the invention.

Preferably, the veterinary composition according to the invention is a liquid and contains at least 0.4 mg/ml of antioxidant, preferably of butylhydroxytoluene, more preferably at least 0.5 mg/ml, still more preferably at least 0.6 mg/ml, yet more preferably at least 0.7 mg/ml, even more preferably at least 0.8 mg/ml, most preferably at least 0.9 mg/ml, and in particular at least 1.0 mg/ml, in each case relative to the total volume of the veterinary composition.

Preferably, the veterinary composition according to the invention is a liquid and contains at most 2.2 mg/ml of antioxidant, preferably of butylhydroxytoluene, more preferably at most 2.0 mg/ml, still more preferably at most 1.8 mg/ml, yet more preferably at most 1.6 mg/ml, even more preferably at most 1.4 mg/ml, most preferably at most 1.2 mg/ml, and in particular at most 1.0 mg/ml, in each case relative to the total volume of the veterinary composition.

Preferably, the veterinary composition according to the invention is a liquid and the content of antioxidant, preferably of butylhydroxytoluene, is within the range of 1.0±0.9 mg/ml of, more preferably of 1.0±0.8 mg/ml, still more preferably of 1.0±0.7 mg/ml, yet more preferably of 1.0±0.6 mg/ml, even more preferably of 1.0±0.5 mg/ml, most preferably of 1.0±0.4 mg/ml, and in particular of 1.0±0.3 mg/ml, in each case relative to the total volume of the veterinary composition.

Suitable solvents include but are not limited to solvents selected from the group consisting of benzyl alcohol, methanol, ethanol, n-propanol, isopropanol, glycerol, acetone, dimethylsulfoxide (DMSO), N,N-dimethylacetamide (DMAC), N,N-dimethylformamide (DMF), and mixtures thereof. Benzyl alcohol is a particularly preferred solvent according to the invention. According to the ATC index 2019, benzyl alcohol (P03AX06) belongs to the subgroup of other ectoparasiticides, incl. scabicides (P03AX), which belongs to the group of ectoparasiticides, incl. scabicides (P03A), which belongs to the class of ectoparasiticides, incl. scabicides, insecticides and repellents (P03). Thus, besides Moxidectin and preferably present Imidacloprid benzyl alcohol also may also contribute to the overall antiparasitic efficacy, potency and efficiency of the veterinary composition according to the invention.

Preferably, the veterinary composition according to the invention is a liquid and contains at least 500 mg/ml of solvent, preferably of benzyl alcohol, more preferably at least 550 mg/ml, still more preferably at least 600 mg/ml, yet more preferably at least 650 mg/ml, even more preferably at least 700 mg/ml, most preferably at least 750 mg/ml, and in particular at least 800 mg/ml, in each case relative to the total volume of the veterinary composition.

Preferably, the veterinary composition according to the invention is a liquid and contains at most 880 mg/ml of solvent, preferably of benzyl alcohol, more preferably at most 870 mg/ml, still more preferably at most 860 mg/ml, yet more preferably at most 850 mg/ml, even more preferably at most 840 mg/ml, most preferably at most 830 mg/ml, and in particular at most 825 mg/ml, in each case relative to the total volume of the veterinary composition.

Preferably, the veterinary composition according to the invention is a liquid and the content of solvent, preferably of benzyl alcohol, is within the range of 815±40 mg/ml of, more preferably of 815±35 mg/ml, still more preferably of 815±30 mg/ml, yet more preferably of 815±25 mg/ml, even more preferably of 815±20 mg/ml, most preferably of 815±15 mg/ml, and in particular of 815±10 mg/ml, in each case relative to the total volume of the veterinary composition.

Suitable co-solvents include but are not limited to co-solvents selected from the group consisting of propylene carbonate, ethylene carbonate, 1,2-butylene carbonate, glycerol carbonate, ethylene glycol, propylene glycol, butyl diglycol, ethylene glycol monomethyl ether, ethylene glycol monoethyl ether, diethylene glycol monoethyl ether, dipropylene glycol monoethyl ether, dipropylene glycol n-butyl ether, and mixtures thereof. Propylene carbonate is a particularly preferred co-solvent according to the invention.

Preferably, the veterinary composition according to the invention is a liquid and contains at least 80 mg/ml of co-solvent, preferably of propylene carbonate, more preferably at least 100 mg/ml, still more preferably at least 120 mg/ml, yet more preferably at least 140 mg/ml, even more preferably at least 160 mg/ml, most preferably at least 165 mg/ml, and in particular at least 800 mg/ml, in each case relative to the total volume of the veterinary composition.

Preferably, the veterinary composition according to the invention is a liquid and contains at most 280 mg/ml of co-solvent, preferably of propylene carbonate, more preferably at most 260 mg/ml, still more preferably at most 240 mg/ml, yet more preferably at most 220 mg/ml, even more preferably at most 200 mg/ml, most preferably at most 180 mg/ml, and in particular at most 165 mg/ml, in each case relative to the total volume of the veterinary composition.

Preferably, the veterinary composition according to the invention is a liquid and the content of co-solvent, preferably of propylene carbonate, is within the range of 165±70 mg/ml of, more preferably of 165±60 mg/ml, still more preferably of 165±50 mg/ml, yet more preferably of 165±40 mg/ml, even more preferably of 165±30 mg/ml, most preferably of 165±20 mg/ml, and in particular of 165±10 mg/ml, in each case relative to the total volume of the veterinary composition.

The veterinary composition according to the invention may optionally contain further excipients, such as carriers, diluents, extenders, solubilizers, surfactants, skin permeation enhancers, fragrances, bitter substances, and the like.

In a particularly preferred embodiment, the veterinary composition according to the invention comprises, preferably essentially consists of
- Moxidectin;
- Imidacloprid;
- triethanolamine;
- optionally, butylhydroxytoluene;
- optionally, benzyl alcohol; and
- optionally, propylene carbonate.

Particularly preferred embodiments of the veterinary composition according to the invention, preferably of the veterinary spot-on solution according to the invention, are compiled as embodiments A¹ to A¹² in the table here below:

| [mg/ml] | A¹ | A² | A³ | A⁴ |
|---|---|---|---|---|
| Imidacloprid, preferably in its non-salt form | 100±90 | 100±70 | 100±50 | 100±30 |
| Moxidectin, preferably in its non-salt form | 17.5±15 | 17.5±13 | 17.5±10 | 17.5±8 |
| solvent, preferably benzyl alcohol | 825±800 | 825±600 | 825±400 | 825±200 |
| co-solvent, preferably propylene carbonate | 165±150 | 165±120 | 165±90 | 165±60 |
| antioxidant, preferably butylhydroxytoluene | 1.0±0.9 | 1.0±0.7 | 1.0±0.5 | 1.0±0.3 |
| amine base, preferably triethanolamine | 0.10±0.09 | 0.10±0.07 | 0.10±0.05 | 0.10±0.03 |

| [mg/ml] | A⁵ | A⁶ | A⁷ | A⁸ |
|---|---|---|---|---|
| Imidacloprid, preferably in its non-salt form | 100±90 | 100±70 | 100±50 | 100±30 |
| Moxidectin, preferably in its non-salt form | 10±9 | 10±7 | 10±5 | 10±3 |
| solvent, preferably benzyl alcohol | 825±800 | 825±600 | 825±400 | 825±200 |
| co-solvent, preferably propylene carbonate | 165±150 | 165±120 | 165±90 | 165±60 |
| antioxidant, preferably butylhydroxytoluene | 1.0±0.9 | 1.0±0.7 | 1.0±0.5 | 1.0±0.3 |
| amine base, preferably triethanolamine | 0.10±0.09 | 0.10±0.07 | 0.10±0.05 | 0.10±0.03 |

| [mg/ml] | A⁹ | A¹⁰ | A¹¹ | A¹² |
|---|---|---|---|---|
| Imidacloprid, preferably in its non-salt form | 100±90 | 100±70 | 100±50 | 100±30 |
| Moxidectin, preferably in its non-salt form | 25±20 | 25±15 | 25±10 | 25±5 |
| solvent, preferably benzyl alcohol | 825±800 | 825±600 | 825±400 | 825±200 |
| co-solvent, preferably propylene carbonate | 165±150 | 165±120 | 165±90 | 165±60 |
| antioxidant, preferably butylhydroxytoluene | 1.0±0.9 | 1.0±0.7 | 1.0±0.5 | 1.0±0.3 |
| amine base, preferably triethanolamine | 0.10±0.09 | 0.10±0.07 | 0.10±0.05 | 0.10±0.03 |

In a particularly preferred embodiment, the veterinary composition according to the invention comprises, preferably essentially consists of
- 7-13 mg/ml of Moxidectin;
- 70 - 130 mg/ml of Imidacloprid;
- 0.07 - 0.13 mg/ml of triethanolamine;
- 0.7 - 1.3 mg/ml of butylhydroxytoluene;
- 625 - 1025 mg/ml of benzyl alcohol; and
- 105 - 225 mg/ml of propylene carbonate.

In a particularly preferred embodiment, the veterinary composition according to the invention comprises, preferably essentially consist of
- 20 - 30 mg/ml of Moxidectin;
- 70 - 130 mg/ml of Imidacloprid;
- 0.07 - 0.13 mg/ml of triethanolamine;
- 0.7 to 1.3 mg/ml of butylhydroxytoluene;
- 625 -1025 mg/ml of benzyl alcohol; and
- 105 - 225 mg/ml of propylene carbonate.

In a particularly preferred embodiment, the veterinary composition according to the invention comprises, preferably essentially consists of
- 10 mg/ml of Moxidectin;
- 100 mg/ml of Imidacloprid;
- 0.11 mg/ml of triethanolamine;
- 1 mg/ml of butylhydroxytoluene;
- 822.19 mg/ml of benzyl alcohol; and
- 165 mg/ml of propylene carbonate.

In a particularly preferred embodiment, the veterinary composition according to the invention comprises, preferably essentially consist of
- 25 mg/ml of Moxidectin;
- 100 mg/ml of Imidacloprid;
- 0.11 mg/ml of triethanolamine;
- 1 mg/ml of butylhydroxytoluene;
- 807.19 mg/ml of benzyl alcohol; and
- 165 mg/ml of propylene carbonate.

The veterinary composition according to the invention is preferably packaged in suitable containers, preferably in unit dose pipettes or other suitable dispensing devices, that are preferably disposable and intended for single use. Unit dose pipettes are preferably equipped with a screw cap. Depending upon the size/weight of the animal to be treated and the corresponding number of spots on which the preferably liquid spot-on solution according to the invention is preferably to be applied, typical volumes of the veterinary composition in a container may range from e.g. about 0.25 ml to 5.0 ml. Preferred containers are unit dose pipettes containing 0.4 ml, or 0.8 ml, or 1.0 ml, or 2.5 ml, or 4.0 ml. A multitude of pipettes may then be packaged e.g. in a blister pack containing e.g. 3 or 6 unit dose pipettes. Preferred unit dose pipettes contain the following volumes of the veterinary composition according to the invention:
spot-on solution for small cats and ferrets (≤ 4 kg body weight): 0.4 ml per pipette;
spot-on solution for large cats (>4 to 8 kg body weight): 0.8 ml per pipette;
spot-on solution for small dogs (≤ 4 kg body weight): 0.4 ml per pipette;
spot-on solution for medium dogs (>4 to 10 kg body weight): 1.0 ml per pipette;
spot-on solution for large dogs (>10 to 25 kg body weight): 2.5 ml per pipette; and
spot-on solution for extra large dogs (>25 to 40 kg body weight): 4.0 ml per pipette.

The veterinary compositions according to the invention can be manufactured by standard procedures of pharmaceutical technology using standard equipment that is commercially available.

For example, when the veterinary composition is a spot-on solution in solvent and co-solvent additionally containing excipients such as antioxidant, it is preferred to firstly mix and dissolve excipients and amine base according to the invention in solvent and co-solvent, and thereafter to add Moxidectin and Imidacloprid. The resultant mixture may then be stirred until all ingredients are fully dissolved. The resultant solution may then be filtered, e.g. through a stainless steel filter. Aliquots of the solution may be filled into pipettes, e.g. made of polypropylene, which may be sealed and packaged, e.g. in laminated triplex bags (PET/Al/LDPE).

Another aspect of the invention relates to the veterinary composition according to the invention as described above for use in the treatment or prevention of parasitic infections in pets. Likewise, another aspect of the invention relates to a veterinary composition of Moxidectin or a physiologically acceptable salt thereof in combination with Imidacloprid or a physiologically acceptable salt thereof, as described above for the treatment or prevention of parasitic infections in pets. Likewise, another aspect of the invention relates to a veterinary composition for use in a method of treating or preventing parasitic infections in pets comprising administering, preferably topically cutaneously, an effective amount of the veterinary composition according to the invention as described above.

Preferably, the pets are selected from cats, ferrets and dogs.

The veterinary composition according to the invention is useful for treating various mixed parasitic infections.

For cats suffering from or at risk of mixed parasitic infections, exemplified mixed parasitic infections that can be treated or prevented with the veterinary composition according to the invention include but are not limited to
- treatment and prevention of flea infestation (*Ctenocephalides felis*);
- treatment of ear mite infestation (*Otodectes cynotis*);
- treatment of notoedric mange (*Notoedres cati*);
- treatment of the lungworm *Eucoleus aerophilus* (*syn. Capillaria aerophila*) (adults);
- prevention of lungworm disease (L3/L4 larvae of *Aelurostrongylus abstrusus*);
- treatment of the lungworm *Aelurostrongylus abstrusus* (adults);
- treatment of the eye worm *Thelazia callipaeda* (adults);
- prevention of heartworm disease (L3 and L4 larvae of *Dirofilaria immitis*); and
- treatment of infections with gastrointestinal nematodes (L4 larvae, immature adults and adults of *Toxocara cati* and *Ancylostoma tubaeforme*).

The veterinary composition according to the invention is preferably used as part of a treatment strategy for flea allergy dermatitis (FAD) in cats.

For ferrets suffering from or at risk of mixed parasitic infections, exemplified mixed parasitic infections that can be treated or prevented with the veterinary composition according to the invention include but are not limited to
- treatment and prevention of flea infestation (*Ctenocephalides felis*); and
- prevention of heartworm disease (L3 and L4 larvae of *Dirofilaria immitis*).

For dogs suffering from or at risk of mixed parasitic infections, exemplified mixed parasitic infections that can be treated or prevented with the veterinary composition according to the invention include but are not limited to
- treatment and prevention of flea infestation (*Ctenocephalides felis*);
- treatment of biting lice (*Trichodectes canis*);
- treatment of ear mite infestation (*Otodectes cynotis*), sarcoptic mange (caused by *Sarcoptes scabiei var. canis*), demodicosis (caused by *Demodex canis*);
- prevention of heartworm disease (L3 and L4 larvae of *Dirofilaria immitis*) and angiostrongylosis (L4 larvae and immature adults of *Angiostrongylus vasorum*);
- treatment of *Angiostrongylus vasorum* and *Crenosoma vulpis*; and
- treatment of infections with gastrointestinal nematodes (L4 larvae, immature adults and adults of *Toxocara canis*, *Ancylostoma caninum* and *Uncinaria stenocephala*, adults of *Toxascaris leonina* and *Trichuris vulpis*).

The veterinary composition according to the invention is preferably used as part of a treatment strategy for flea allergy dermatitis (FAD) in dogs.

Preferably, the veterinary composition according to the invention is administered topically to the skin, preferably to undamaged skin.

Preferably, the veterinary composition according to the invention is administered cutaneously by applying it in form of a spot-on composition, preferably spot-on solution to the skin. Depending upon the size and weight of the animal to be treated, the spot-on composition is to be applied, e.g. pipetted, to a single spot of skin or to several spots of skin, typically to 1, 2, 3 or 4 spots.

The recommended minimum dose of Imidacloprid is 10 mg per kg body weight. When the concentration of Imidacloprid in the veterinary solution is e.g. 100 mg/ml, this corresponds to 0.1 ml per kg body weight. The recommended minimum dose of Moxidectin then corresponds to the amount of Moxidectin that is contained in this volume of the veterinary composition as calculated for Imidacloprid.

Preferred doses of Imidacloprid and Moxidectin are also compiled in the table here below:

| mg per kg body weight | body weight | Imidacloprid | Moxidectin |
|---|---|---|---|
| Ferrets | all | minimum 10 | minimum 1.0 |
| Cats | ≤ 4 kg | minimum 10 | minimum 1.0 |
| | > 4 to 8 kg | 10 to 20 | 1.0 to 2.0 |
| Dogs | ≤ 4 kg | minimum 10 | minimum 2.5 |
| | > 4 to 10 kg | 10 to 25 | 2.5 to 6.25 |
| | > 10 to 25 kg | 10 to 25 | 2.5 to 6.25 |
| | > 25 to 40 kg | 10 to 16 | 2.5 to 4.0 |

Preferably, the veterinary composition according to the invention is administered at least once.

In a preferred embodiment, an effective amount of the veterinary composition according to the invention is administered a single time and then treatment is terminated.

In another preferred embodiment, a first effective amount of the veterinary composition according to the invention is administered, i.e. applied to the skin, and after 2 weeks, 3 weeks or 4 weeks, a second effective amount of the veterinary composition according to the invention is administered, i.e. applied to the skin. After administration of the second effective amount, treatment may be terminated.

Alternatively, after administration of the second effective amount, treatment may be continued, i.e. after 2 weeks, 3 weeks or 4 weeks, a third effective amount of the veterinary composition according to the invention is administered, i.e. applied to the skin. After administration of the third effective amount, treatment may be terminated or continued.

When treatment involves repeated administration, every administered subsequent effective amount of the veterinary composition according to the invention preferably essentially corresponds to every administered preceding effective amount of the veterinary composition according to the invention (i.e. first effective dose ≈ second effective dose ≈ third effective dose and so on).

Another aspect relates to the use of an amine base according to the invention as described above, triethanolamine, diethanolamine, monoethanolamine, N-methyldiethanolamine, N-ethyldiethanolamine, N,N-dimethylethanolamine, or N,N-diethylethanolamine, preferably triethanolamine, for stabilizing Moxidectin or a physiologically acceptable salt thereof. Preferably, Moxidectin is stabilized against chemical decomposition, preferably in solution.

Another aspect relates to the use of an amine base according to the invention as described above, triethanolamine, diethanolamine, monoethanolamine, N-methyldiethanolamine, N-ethyldiethanolamine, N,N-dimethylethanolamine, or N,N-diethylethanolamine, preferably triethanolamine, for producing a veterinary composition with improved stability of Moxidectin or a physiologically acceptable salt thereof, preferably for producing a veterinary composition according to the invention as described above.

The following examples further illustrate the invention but are not to be construed as limiting its scope.

### Examples 1 and 2:

The following veterinary compositions were prepared:

| Imidacloprid/Moxidectin spot-on solution | | |
|---|---|---|
| | Example 1 | Example 2 |
| Strength | 100 mg/10 mg/ml | 100 mg/25 mg/ml |
| Ingredient | Quantity/unit [mg/ml] | Quantity/unit [mg/ml] |
| Imidacloprid | 100.00 | 100.00 |
| Moxidectin | 10.00 | 25.00 |
| Propylene Carbonate | 165.00 | 165.00 |
| Butylhydroxytoluene | 1.00 | 1.00 |
| Triethanolamine | 0.11* | 0.11* |
| Benzyl Alcohol | 822.19 | 807.19 |

| | | |
|---|---|---|
| * 0.11 mg/ml of triethanolamine corresponds to 100 ppm of triethanolamine | | |

After mixing and dissolving of the excipients benzyl alcohol, propylene carbonate, butylhydroxytoluene and triethanolamine, the active ingredients are added and the solution is mixed until active ingredients are fully dissolved.

As the final step of manufacturing process filtration through stainless steel filter is performed to remove possible mechanical particles.

The solution is filled into polypropylene pipette. Pipettes are sealed and put into the laminated triplex bags (PET/Al/LDPE).

### Example 3:

Four different spot-on solutions according to Examples 1 (I-1 and I-2) and 2 (I-3 and I-4) were prepared containing Imidacloprid and Moxidectin in the same concentrations as the reference product (Advocate^{®}) and the same excipients (butylhydroxytoluene, benzyl alcohol, propylene carbonate), but additionally containing triethanolamine (TEA).

Each spot-on solution was filled into polypropylene pipette and the pipettes were stored in aluminum triplex bags under accelerated storage conditions (6 months at 40°C and 75% r.h.).

Two degradation products were evaluated by HPLC before and after storage, the isomer of Moxidectin eluting at approx. relative retention time 1.15, formed due to oxidation of moxidectin, and 23-keto-nemadectin, eluting at approx. relative retention time 0.43, a hydrolytic degradation product (column with octadecylsilyl stationary phase, 150 mm x 4.6 mm i.d., 2.6 µm particles, column temperature 50 °C, isocratic elution of 0.25M ammonium acetate pH 4.6 and acetonitrile in ratio 40 : 60 (V/V) and flow rate 1.5 mL/min, UV detection at 242 nm and injection volume 10 µL; solvent acetonitrile).

The results are compiled in the following table:

| Example * | | I-1 | I-2 | Advocate^{®} (cats) | I-3 | I-4 | Advocate^{®} (dogs) |
|---|---|---|---|---|---|---|---|
| Volume of solution filled in pipette (ml) | | 0.4 | 0.8 | 0.4 | 0.4 | 2.5 | 0.4 |
| Imidacloprid \|mg/ml\| | | 100 | 100 | 100 | 100 | 100 | 100 |
| Moxidectin [mg/ml] | | 10 | 10 | 10 | 25 | 25 | 25 |
| Triethanolamine [ppm] | | 100 | 100 | - | 100 | 100 | - |
| 23-keto-nemadectin | before storage | ≤0.05 | ≤0.05 | 0.15 | ≤0.05 | ≤0.05 | 1.03 |
| | after storage | ≤0.05 | ≤0.05 | 1.66 | ≤0.05 | ≤0.05 | 5.74 |
| | relative change | - | - | 1.51 | - | - | 4.71 |
| isomer of Moxidectin | before storage | 0.07 | 0.07 | 0.05 | 0.08 | 0.08 | 0.11 |
| | after storage | 0.11 | 0.11 | 0.15 | 0.16 | 0.16 | 0.46 |
| | relative change | 0.04 | 0.04 | 0.10 | 0.08 | 0.08 | 0.35 |

* All formulations including comparative product Advocate^{®} additionally contained benzyl alcohol (solvent), propylene carbonate (co-solvent), and butylhydroxytoluene (antioxidant).

As demonstrated by the above comparative data, the veterinary compositions according to the invention containing amine base triethanolamine have a significantly improved storage stability compared to commercial Advocate^{®} product not containing amine base.

### Example 4:

Two spot-on solutions A and B were prepared according to Examples 7 and 8 of WO 2006/0508(not according to the invention):

| Product | Imidacloprid/Moxidectin spot-on solution | |
|---|---|---|
| Strength | 100 mg/10 mg/ml | 100 mg/25 mg/ml |
| | Example 4A | Example 4B |
| Ingredient | Quantity/unit [mg/ml] | Quantity/unit [mg/ml] |
| Imidacloprid | 100 | 100 |
| Moxidectin | 10 | 25 |
| Propylene carbonate | 165.0 | 165.0 |
| Butylhydroxytoluene | 1 | 1 |
| Benzyl alcohol | 822.3 | 807.3 |

Each spot-on solution was filled into polypropylene pipette and the pipettes were stored in aluminum triplex bags under accelerated storage conditions (3 or 6 months at 40°C and 75% r.h.) and long-term conditions (12 or 24 months at 25°C and 60% r.h.).

The stability test were additionally performed by preparing inventive formulations containing Moxidectin obtained from different sources I (I-1, I-2, I-3 and I-4) and II (I-5 and I-6).

The results are compiled in the following tables:

| Examples (strength 100 mg/10 mg/ml) | | I-1 | I-2 | I-5 | 4A * |
|---|---|---|---|---|---|
| Imidacloprid [mg/ml] | | 100 mg | 100 mg | 100 mg | 100 mg |
| Moxidectin [mg/ml] | | 10 mg | 10 mg | 10 mg | 10 mg |
| Moxidectin source | | I | I | II | I |
| Triethanolamine [ppm] | | 100 | 100 | 100 | - |
| 23-keto-nemadectin | initial | ≤ 0.05 | ≤ 0.05 | <0.05 | ≤ 0.05 |
| | 3m 40°C/75%RH | ≤ 0.05 | ≤ 0.05 | <0.05 | 1.44 |
| | 6m 40°C/75%RH | ≤ 0.05 | ≤ 0.05 | 0.11 | n.a. |
| | 12m 25°C/60%RH | ≤ 0.05 | ≤ 0.05 | n.a. | 0.80 |
| | 24m 25°C/60%RH | ≤ 0.05 | ≤ 0.05 | n.a. | n.a. |
| isomer of moxidectin | initial | 0.07 | 0.07 | 0.05 | 0.07 |
| | 3m 40°C/75%RH | 0.07 | 0.08 | 0.06 | 1.26 |
| | 6m 40°C/75%RH | 0.11 | 0.11 | 0.07 | n.a. |
| | 12m 25°C/60%RH | 0.07 | 0.07 | n.a. | 0.61 |
| | 24m 25°C/60%RH | 0.10 | 0.10 | n.a. | n.a. |

| Examples (strength 100 mg/25 mg/ml) | I-3 | I-4 | I-6 | 4B ∗ | |
|---|---|---|---|---|---|
| Imidacloprid [mg/ml] | 100 mg | 100 mg | 100 mg | 100 mg | |
| Moxidectin [mg/ml] | 25 mg | 25 mg | 25 mg | 25 mg | |
| Moxidectin source | I | I | II | I | |
| Triethanolar mine [ppm] | | 100 | 100 | 100 | - |
| 23-keto-nemadectin | initial | ≤ 0.05 | ≤ 0.05 | <0.05 | ≤ 0.05 |
| | 3m 40°C/75%RH | ≤ 0.05 | ≤ 0.05 | <0.05 | 0.32 |
| | 5m 40°C/75%RH | ≤ 0.05 | ≤ 0.05 | <0.05 | 1.89 |
| | 12m 25°C/60%RH | ≤ 0.05 | ≤ 0.05 | n.a. | n.a. |
| | 24m 25°C/60%RH | ≤ 0.05 | ≤ 0.05 | n.a. | n.a. |
| isomer of moxidectin | initial | 0.08 | 0.08 | <0.05 | 0.12 |
| | 3m 40°C/75%RH | 0.11 | 0.11 | 0.06 | 0.20 |
| | 5m 40°C/75%RH | 0.16 | 0.16 | 0.07 | 0.87 |
| | 12m 25°C/60%RH | 0.09 | 0.09 | n.a. | n.a. |
| | 24m 25°C/60%RH | 0.12 | 0.12 | n.a. | n.a. |

| | | | | | |
|---|---|---|---|---|---|
| n.a. - not analyzed * example 4A and 4B are reference examples | | | | | |

As demonstrated by the above comparative stability data, the veterinary compositions according to the invention containing amine base triethanolamine have a significantly improved storage stability compared to compositions disclosed in prior art document not containing amine base. The data show that the amount of impurities that formed in the veterinary compositions according to the invention containing amine base triethanolamine is significantly lower than in the compositions disclosed in prior art document not containing amine base. The increase in the amount of impurities in the veterinary compositions according to the invention is minimal, whereas in the prior art compositions the amount of impurities that formed already in the first year after subjecting to long term storage conditions significantly increased.

Furthermore, by stability testing of the veterinary compositions according to the invention comprising different sources of Moxidectin (I and II) it has been shown that the formulation is robust and the quality of Moxidectin does not influence the stability of the compositions.

## Claims

1. A veterinary composition for cutaneous topical application comprising
(i) Moxidectin or a physiologically acceptable salt thereof;
(ii) Imidacloprid or a physiologically acceptable salt thereof; and
(iii) a physiologically acceptable amine base selected from the group consisting of triethanolamine, diethanolamine, monoethanolamine, N-methyldiethanolamine, N-ethyldiethanolamine, N,N-dimethylethanolamine, and N,N-diethylethanolamine;
wherein the weight content of the amine base is within the range of from 1 ppm to 1000 ppm, relative to the total weight of the veterinary composition.

2. The veterinary composition according to any of the preceding claims, wherein the protonated form of the amine base has a pK_{A} value within the range of from 7.20 to 10.00; preferably from 7.20 to 9.50; more preferably from 7.20 to 9.00, still more preferably from 7.20 to 8.50.

3. The veterinary composition according to any of the preceding claims, wherein the amine base is triethanolamine.

4. The veterinary composition according to any of the preceding claims, wherein the weight content of the amine base is at most 240 ppm, relative to the total weight of the veterinary composition; preferably at most 230 ppm, more preferably at most 220 ppm, still more preferably at most 210 ppm, yet more preferably at most 200 ppm, even more preferably at most 190 ppm, and most preferably at most 180 ppm.

5. The veterinary composition according to any of the preceding claims, which
- is a liquid; and/or
- is a solution, suspension or emulsion; and/or
- is anhydrous.

6. The veterinary composition according to any of the preceding claims, which additionally contains one or more excipients selected from antioxidants, preservatives, solvents, and co-solvents.

7. The veterinary composition according to any of the preceding claims, which additionally contains an antioxidant selected from the group consisting of di-tert-butylhydroxytoluene (BHT), butylhydroxyanisole (BHA), alpha-tocopherol, alpha-tocopheryl acetate, ascorbic acid, salts of ascorbic acid, monothioglycerol, phosphorous acid, coniferyl benzoate, nordihydro-guajaretic acid, gallus acid esters, sodium bisulfite, and mixtures thereof.

8. The veterinary composition according to any of the preceding claims, which additionally contains a solvent selected from the group consisting of benzyl alcohol, methanol, ethanol, n-propanol, isopropanol, glycerol, acetone, dimethylsulfoxide, N,N-dimethylacetamide, N,N-dimethylformamide, and mixtures thereof.

9. The veterinary composition according to any of the preceding claims, which additionally contains a co-solvent selected from the group consisting of propylene carbonate, ethylene carbonate, 1,2-butylene carbonate, glycerol carbonate, ethylene glycol, propylene glycol, butyl diglycol, ethylene glycol monomethyl ether, ethylene glycol monoethyl ether, diethylene glycol monoethyl ether, dipropylene glycol monoethyl ether, dipropylene glycol n-butyl ether, and mixtures thereof.

10. The veterinary composition according to any of the preceding claims, which comprises
- Moxidectin;
- Imidacloprid;
- triethanolamine;
- optionally, butylhydroxytoluene;
- optionally, benzyl alcohol; and
- optionally, propylene carbonate.

11. The veterinary composition according to any of the preceding claims for use in the treatment or prevention of parasitic infections in pets.

12. The veterinary composition for use according to claim 11, wherein the pets are selected from cats, ferrets and dogs.

13. The veterinary composition for use according to claim 11 or 12, wherein the veterinary composition is administered topically to the skin.

## Patentansprüche

1. Veterinärmedizinische Zusammensetzung zur topischen Anwendung auf der Haut, umfassend
(i) Moxidectin oder dessen physiologisch verträgliches Salz;
(ii) Imidacloprid oder dessen physiologisch verträgliches Salz; und
(iii) eine physiologisch verträgliche Aminbase, die ausgewählt wird aus der Gruppe bestehend aus Triethanolamin, Diethanolamin, Monoethanolamin, N-Methyldiethanolamin, N-Ethyl-diethanolamin, N,N-Dimethyl-ethanolamin und N, N-Diethyl-ethanolamin;
wobei der Gewichtsgehalt der Aminbase im Bereich von 1 ppm bis 1000 ppm, bezogen auf das Gesamtgewicht der veterinärmedizinischen Zusammensetzung, liegt.

2. Die veterinärmedizinische Zusammensetzung gemäß einem der vorhergehenden Ansprüche, wobei die protonierte Form der Aminbase einen pK_{A}-Wert im Bereich von 7,20 bis 10,00, bevorzugt von 7,20 bis 9,50; besonders bevorzugt von 7,20 bis 9,00, noch weiter bevorzugt von 7,20 bis 8,50, aufweist.

3. Die veterinärmedizinische Zusammensetzung gemäß einem der vorhergehenden Ansprüche, wobei die Aminbase Triethanolamin ist.

4. Die veterinärmedizinische Zusammensetzung gemäß einem der vorhergehenden Ansprüche, wobei der Gewichtsgehalt der Aminbase höchstens 240 ppm, bezogen auf das Gesamtgewicht der veterinärmedizinischen Zusammensetzung, beträgt; bevorzugt höchstens 230 ppm, besonders bevorzugt höchstens 220 ppm, noch weiter bevorzugt höchstens 210 ppm, noch weiter bevorzugt höchstens 200 ppm, ganz besonders bevorzugt höchstens 190 ppm und am meisten bevorzugt höchstens 180 ppm.

5. Die veterinärmedizinische Zusammensetzung gemäß einem der vorhergehenden Ansprüche, die
- eine Flüssigkeit ist; und/oder
- eine Lösung, Suspension oder Emulsion ist; und/oder
- wasserfrei ist.

6. Die veterinärmedizinische Zusammensetzung gemäß einem der vorhergehenden Ansprüche, die zusätzlich einen oder mehrere Hilfsstoffe, ausgewählt aus Antioxidantien, Konservierungsmitteln, Lösungsmitteln und Co-Lösungsmitteln, enthält.

7. Die veterinärmedizinische Zusammensetzung gemäß einem der vorhergehenden Ansprüche, die zusätzlich ein Antioxidationsmittel enthält, das ausgewählt wird aus der Gruppe bestehend aus Di-tert-butylhydroxytoluol (BHT), Butylhydroxyanisol (BHA), alpha-Tocopherol, alpha-Tocopherylacetat, Ascorbinsäure, Salze der Ascorbinsäure, Monothioglycerin, phosphorige Säure, Coniferylbenzoat, Nordihydroguajaretinsäure, Gallussäureester, Natriumbisulfit und Mischungen davon.

8. Die veterinärmedizinische Zusammensetzung gemäß einem der vorhergehenden Ansprüche, die zusätzlich ein Lösungsmittel enthält, das ausgewählt wird aus der Gruppe bestehend aus Benzylalkohol, Methanol, Ethanol, n-Propanol, Isopropanol, Glycerin, Aceton, Dimethylsulfoxid, N,N-Dimethylacetamid, N,N-Dimethylformamid und Mischungen davon.

9. Die veterinärmedizinische Zusammensetzung gemäß einem der vorhergehenden Ansprüche, die zusätzlich ein Co-Lösungsmittel enthält, das ausgewählt wird aus der Gruppe bestehend aus Propylencarbonat, Ethylencarbonat, 1,2-Butylencarbonat, Glycerincarbonat, Ethylenglykol, Propylenglykol, Butyldiglykol, Ethylenglykolmonomethylether, Ethylenglykolmonoethylether, Diethylenglykolmonoethylether, Dipropylenglykolmonoethylether, Dipropylenglykol-n-butylether und deren Mischungen.

10. Die veterinärmedizinische Zusammensetzung gemäß einem der vorhergehenden Ansprüche, die Folgendes umfasst:
- Moxidectin;
- Imidacloprid;
- Triethanolamin;
- gegebenenfalls Butylhydroxytoluol;
- gegebenenfalls Benzylalkohol; und
- gegebenenfalls Propylencarbonat.

11. Die veterinärmedizinische Zusammensetzung gemäß einem der vorhergehenden Ansprüche zur Verwendung bei der Behandlung oder Vorbeugung von parasitären Infektionen bei Haustieren.

12. Die veterinärmedizinische Zusammensetzung zur Verwendung gemäß Anspruch 11, wobei die Haustiere aus Katzen, Frettchen und Hunden ausgewählt werden.

13. Die veterinärmedizinische Zusammensetzung zur Verwendung gemäß Anspruch 11 oder 12, wobei die veterinärmedizinische Zusammensetzung topisch auf die Haut verabreicht wird.

## Revendications

1. Composition vétérinaire destinée à une application topique cutanée comprenant
(i) de la moxidectine ou un sel physiologiquement acceptable de celle-ci ;
(ii) de l'imidaclopride ou un sel physiologiquement acceptable de celui-ci ; et
(iii) une base d'amine physiologiquement acceptable choisie dans le groupe constitué par la triéthanolamine, la diéthanolamine, la monoéthanolamine, la N-méthyldiéthanolamine, la N-éthyldiéthanolamine, la N,N-diméthyléthanolamine et la N,N-diéthyléthanolamine ;
dans laquelle la teneur en poids de la base d'amine est dans la plage de 1 ppm à 1000 ppm, par rapport au poids total de la composition vétérinaire.

2. Composition vétérinaire selon l'une quelconque des revendications précédentes, dans laquelle la forme protonée de la base d'amine a une valeur de pK_{A} dans la plage de 7,20 à 10,00 ; de préférence de 7,20 à 9,50 ; de manière davantage préférée de 7,20 à 9,00, de manière encore davantage préférée de 7,20 à 8,50.

3. Composition vétérinaire selon l'une quelconque des revendications précédentes, dans laquelle la base d'amine est la triéthanolamine.

4. Composition vétérinaire selon l'une quelconque des revendications précédentes, dans laquelle la teneur en poids de base d'amine est d'au plus 240 ppm, par rapport au poids total de la composition vétérinaire ; de préférence d'au plus 230 ppm, de manière davantage préférée d'au plus 220 ppm, de manière encore davantage préférée d'au plus 210 ppm, de manière encore davantage préférée d'au plus 200 ppm, de manière encore davantage préférée d'au plus 190 ppm, et de manière préférée entre toutes d'au plus 180 ppm.

5. Composition vétérinaire selon l'une quelconque des revendications précédentes, qui
- est un liquide ; et/ou
- est une solution, une suspension ou une émulsion ; et/ou
- est anhydre.

6. Composition vétérinaire selon l'une quelconque des revendications précédentes, qui contient en outre un ou plusieurs excipients choisis parmi des antioxydants, des conservateurs, des solvants et des co-solvants.

7. Composition vétérinaire selon l'une quelconque des revendications précédentes, qui contient en outre un antioxydant choisi dans le groupe constitué par le di-tert-butylhydroxytoluène (BHT), le butylhydroxyanisole (BHA), l'alpha-tocophérol, l'acétate d'alpha-tocophéryle, l'acide ascorbique, les sels d'acide ascorbique, le monothioglycérol, l'acide phosphoreux, le benzoate de coniféryle, l'acide nordihydro-guajarétique, les esters d'acide gallique, le bisulfite de sodium et des mélanges de ceux-ci.

8. Composition vétérinaire selon l'une quelconque des revendications précédentes, qui contient en outre un solvant choisi dans le groupe constitué par l'alcool benzylique, le méthanol, l'éthanol, le n-propanol, l'isopropanol, le glycérol, l'acétone, le diméthylsulfoxyde, le N,N-diméthylacétamide, le N,N-diméthylformamide et des mélanges de ceux-ci.

9. Composition vétérinaire selon l'une quelconque des revendications précédentes, qui contient en outre un co-solvant choisi dans le groupe constitué par le carbonate de propylène, le carbonate d'éthylène, le carbonate de 1,2-butylène, le carbonate de glycérol, l'éthylène glycol, le propylène glycol, le butyldiglycol, l'éther monométhylique d'éthylène glycol, l'éther monoéthylique d'éthylène glycol, l'éther monoéthylique de diéthylène glycol, l'éther monoéthylique de dipropylène glycol, l'éther n-butylique de dipropylène glycol et des mélanges de ceux-ci.

10. Composition vétérinaire selon l'une quelconque des revendications précédentes, qui comprend
- de la moxidectine ;
- de l'imidaclopride ;
- de la triéthanolamine ;
- éventuellement, du butylhydroxytoluène ;
- éventuellement, de l'alcool benzylique ; et
- éventuellement du carbonate de propylène.

11. Composition vétérinaire selon l'une quelconque des revendications précédentes destinée à être utilisée dans le traitement ou la prévention d'infections parasitaires chez les animaux de compagnie.

12. Composition vétérinaire destinée à être utilisée selon la revendication 11, dans laquelle les animaux de compagnie sont choisis parmi les chats, les furets et les chiens.

13. Composition vétérinaire destinée à être utilisée selon la revendication 11 ou 12, dans laquelle la composition vétérinaire est administrée par voie topique sur la peau.
